# EUROPEAN PATENT APPLICATION

(11) **EP 1 612 542 A1**
(43) Date of publication of application: **04.01.2006**
(21) Application number: 03816601.3
(22) Date of filing: 07.04.2003
(51) Int. Cl.: G01N 21/78

(54) **METHOD OF MEASURING FRESHNESS OF CEREALS AND BEANS AND APPRATUS THEREFOR**

(71) Applicant: SATAKE CORPORATION, Chiyoda-ku, Tokyo 101-0021 (JP)
(72) Inventor: MIKAMI, Takashi, Chiyoda-ku, Tokyo 101-0021 (JP); KAWAKAMI, Koji, Chiyoda-ku, Tokyo 101-0021 (JP); OCHI, Tatsuhiko, Chiyoda-ku, Tokyo 101-0021 (JP)
(74) Representative: Billington, Lawrence Emlyn
(86) International application number: PCT/JP2003/004405
(87) International publication number: WO 2004/090519

(57) **Abstract**

Grains are put into a test tube containing a specific reagent solution and are stirred to produce a reactive solution and this solution is put into a cell. A light is irradiated to the cell to detect an absorbance at a wavelength where the absorbance reaches a peak and an absorbance in a wavelength region in which the absorbance hardly varies. Then, freshness of the grains and beans is judged based on a difference between the two absorbances.

## Description

### TECHNICAL FIELD

The present invention relates to a method and an apparatus for measuring freshness of grains such as rice, wheat, barley, and corn and beans such as soybeans and coffee beans (unroasted beans) and particularly to a method and an apparatus for measuring the freshness of the grains and beans from a color change of a specific reagent solution in which the grains and beans are put and stirred.

### BACKGROUND TECHNIQUE

Grains include fats such as neutral lipid, bran lipid, and phospholipid. These fats (they are mostly neutral lipid) start deteriorating after harvest, undergoes oxidation, and decompose to produce free fatty acid. This fatty acid further decomposes to become hexanal and the like. Because amounts of the produced fatty acid and hexanal increase in process of time, freshness of the grains can be measured by measuring the amount of the fatty acid.

Conventionally, there is a known method for measuring freshness of rice grains by putting the rice grains into a specific reagent solution using bromothymol blue (BTB) as an indicator, for example, stirring them, and judging a color change of the reagent solution (an amount of fatty acid).

On the other hand, there is a known method for measuring a degree of deterioration of an oil sample such as oil used for frying by putting the oil sample into a reagent solution and stirring it to obtain a reactive solution, by measuring an absorbance of the reactive solution to which a light of a specific wavelength is irradiated and then judging a color change (an amount of fatty acid) based on the absorbance (Japanese Patent Application Laid-open No. 52-32396).

In the former method for measuring the freshness of the rice grains, the color change of the reagent solution is judged visually and therefore judgement on the color differs from person to person and the freshness cannot be judged accurately. Therefore, as the above-mentioned Japanese Patent Application Laid-open No. 52-32396 teaches, the absorbance of a reactive solution to which a light of a specific wavelength is irradiated may be measured to judge the color change of the reactive solution based on the measured absorbance.

However, it cannot be said that accuracy of the freshness judgment of the grains is enhanced by this method. It is possible to judge the freshness of the grains by detecting that the reagent solution has changed color in the presence of fatty acid or hexanal in the grains by measuring the absorbance. However, the reactive solution obtained by putting the grains into the reagent solution and stirring them includes fine particles of the grains (skin, bran, starch, and the like which have been peeled off) and the fine particles make the reactive solution turbid to affect the absorbance.

### DISCLOSURE OF THE INVENTION

It is an object of the present invention to provide a method for accurately measuring freshness of grains and beans even if a reactive solution is turbid due to skin, bran, starch, and others adhering to the grains and beans.

To achieve the above object, according to the invention, there is provided a method of measuring freshness of grains and beans which comprises steps of: putting grains or beans which are targets of the measurement into a predetermined reagent solution and stirring the solution; detecting a first absorbance of a reactive solution obtained at the stirring step, to which a light is irradiated, at a wavelength where an absorbance thereof reaches a peak, and a second absorbance in a wavelength region in which an absorbance hardly varies; and obtaining a difference between the first and second absorbances detected at the absorbance detecting step and judging the freshness of the grains or beans based on the difference.

In the invention, the absorbance (first absorbance) of a reactive solution, to which a light is irradiated, at a wavelength where an absorbance thereof reaches the peak and the absorbance (second absorbance) in the wavelength region in which an absorbance hardly varies are detected, paying attention to the fact that a relationship between a wavelength of irradiated light and an absorbance of the reactive solution is as follows:
(1) The absorbance tends to increase generally at every wavelength as turbidity of a reactive solution increases;
(2) The absorbance tends to decrease generally at every wavelength as turbidity of a reactive solution decreases; and
(3) The wavelength where an absorbance reaches the peak hardly varies irrespective of the level of turbidity of a reactive solution, and there exists a wavelength region in which the absorbance hardly varies (that is, generally absorbances change in a similar way), though the entire level thereof is different dependent on the degree of turbidity. Then, the difference between the first and second absorbances is obtained. The larger the difference, the higher the freshness is judged. Therefore, with the method of the invention, it is possible to accurately judge the freshness irrespective of the degree of turbidity of the reactive solution.

### BRIEF DESCRIPTION OF THE DRAWINGS

FIG. 1 shows an apparatus for measuring freshness of rice grains according to the present invention.
FIG. 2 is a graph showing absorbances of respective reactive solutions.

### BEST MODE FOR CARRYING OUT THE INVENTION

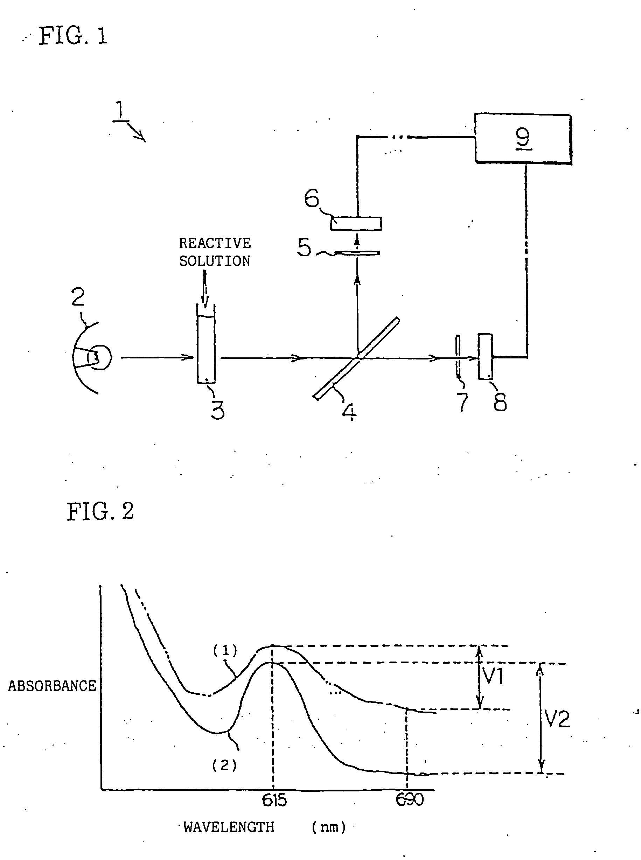

An example of freshness measurement device 1 for grains and beans according to the present invention will be described with reference to FIG. 1. The freshness measurement device 1 includes a light source 2, a cell 3, a half mirror 4, a first filter 5, a first photoreceptor 6, a second filter 7, and a second photoreceptor 8. The cell 3 contains a reactive solution for measurement. The light source 2 applies at least lights of wavelengths of 610nm to 620nm and 680nm or longer to the reactive solution. Then, the light which has passed through the cell 3 is divided by the half mirror 4 into two routes: one toward the first photoreceptor 6 via the first filter 5 and the other toward the second photoreceptor 8 via the second filter 7.

In the present embodiment, the first filter 5 allows a light of a wavelength of 615nm to pass through itself and the second filter 7 allows a light of a wavelength of 690nm to pass through itself. These first and second photoreceptors 6 and 8 are respectively connected to an arithmetic processing means 9 through amplifiers and A/D converters for converting analog signals into digital signals (not shown). This arithmetic processing means 9 mainly includes, for example, a central processing unit (CPU) and also includes input/output portions, a read-only memory (ROM), and read/write memory (RAM). The ROM includes a program for measuring freshness of grains and beans based on signals detected by the first and second photoreceptors 6 and 8.

An outline of the program is as follows.

The detected signals are converted into values as absorbances and a difference (difference value) between the value on the first photoreceptor side and the value on the second photoreceptor side is obtained and stored in the RAM. Then, difference values in a freshness judgment table which has been set and stored in advance in the RAM and the difference value based on the measurement are compared with each other, a closest approximate value is selected, and a judgment value corresponding to the difference value is displayed as a measurement result (measurement value). The freshness judgment table is formed by dividing a possible range of the difference values into several levels and each the level is brought into correspondence with a judgment value such as excellent freshness, good freshness, and passable freshness.

It is also possible that values on the first photoreceptor side and values on the second photoreceptor side are displayed as they are so that they can be contrasted with each other and the values are used as measurement values.

A method of measuring freshness of the grains and beans by using the freshness measurement device 1 in FIG. 1 will be described below.

A reagent solution using bromothymol blue (BTB) as an indicator is employed. An undiluted solution of this reagent solution is prepared by dissolving 0.3g of bromothymol blue in a solution obtained by adding 50ml of distilled water to 150ml of ethanol. This undiluted solution is diluted with distilled water 1 in 30 and an aqueous solution of potassium hydroxide of 0.2% is added to the solution to adjust pH to 7.0. The bromothymol blue in the reagent solution changes color in the presence of fatty acid and hexanal in a sample solution and the color change is not affected by the number of fine particles such as skin, bran, and starch which cause turbidity.

The indicator, BTB, is a pH indicator and does not react with fatty acid and hexanal in the reactive solution. If much fatty acid or hexanal is included in the reactive solution, a pH is low, the reactive solution turns yellow, and absorbance is low. On the other hand, if there is a small amount of fatty acid or hexanal included in the reactive solution, the pH is high, the solution turns blue, and the absorbance is high.

White rice (sample rice grains) of a variety, "Nipponbare" grown in Shiga Prefecture, Japan, is put into a test tube containing this reagent solution and a stopper is fitted in the test tube. The test tube is laid in a shaking machine and shaken 150 times a minute (stirring process). The reagent solution (reactive solution) in the test tube and obtained by this stirring process is put into the cell 3 and a light from the light source 2 is irradiated to the cell 3.

The light which has passed through the cell 3 is divided by the half mirror 4 into two routes. In one route, the light of the wavelength of 615nm passes through the first filter 5 and is received by the first photoreceptor 6. In the other route, the light of the wavelength of 690nm passes through the second filter 7 and is received by the second photoreceptor 8. Then, a voltage signal according to an amount of light received by the first photoreceptor 6 is output from the first photoreceptor 6 and is input to the arithmetic processing means 9 as a signal A1 via the amplifier and the A/C converter. A voltage signal according to an amount of light received by the second photoreceptor 8 is output from the second photoreceptor 8 and is input to the arithmetic processing means 9 as a signal A2 via the amplifier and the A/C converter.

The arithmetic processing means 9 performs an operation, log A2-log A1, based on the input signals A1 and A2 to obtain a difference (absorbance difference) between a first absorbance at the wavelength of 615nm and a second absorbance at the wavelength of 690nm absorbed by the reactive solution in the cell 3.

Here, graphs showing an absorbance of every wavelength with different turbidities of the reactive solution will be described with reference to FIG. 2.

In FIG. 2, a graph (1) shows the absorbance detected from a reactive solution of a high turbidity and a graph (2) shows the absorbance detected from a reactive solution of a low turbidity. As can be understood from FIG. 2, the absorbance reaches a peak at the wavelength of 615nm and levels off at the wavelengths of 690nm and greater in each of the graphs (1) and (2). In other words, it can be understood from the graphs (1) and (2) that the absorbance tends to vary substantially in the same way with respect to the wavelength irrespective of the turbidity of the reactive solution, though the absorbance with respect to wavelength differs dependent upon the turbidity of the reactive solution.

The invention has been made by paying attention to the fact that the absorbance of a reactive solution to which a light is irradiated tends to change generally at every wavelength irrespective of the turbidity of the reactive solution and that, for every cases, there exists a wavelength region in which the absorbance hardly varies in spite of variation in the wavelength. A difference between the absorbance (first absorbance) at the wavelength of 615nm at which the absorbance reaches the peak and the absorbance (second absorbance) at 690nm in the wavelength region in which the absorbance hardly varies is obtained. The larger the difference between the first and second absorbances, the higher the freshness of the rice grain is judged. Therefore, it is possible to judge the freshness of the rice grain without being affected by the turbidity of the reactive solution. The higher the freshness of the rice grain which is a target of measurement, the higher the first absorbance is. On the other hand, the second absorbance appears independent of the freshness of the rice grain. Therefore, it can be said that the freshness of the target rice grain increases as the difference between the first absorbance and the second absorbance increases

To explain by using the example in FIG. 2, a difference between first and second absorbances on the graph (1) is V1 and a difference between first and second absorbances on the graph (2) is V2. In this drawing, because V1 < V2, the freshness of the sample rice grains of the graph (2) can be judged higher than that of the sample rice grains of the graph (1).

When the reagent solution using bromothymol blue (BTB) as the indicator is employed as described above, a wavelength in a range of 610nm to 620nm may be used to obtain the first absorbance and a wavelength of 680nm or longer may be used from the same light source to obtain the second absorbance.

Although the reagent solution using bromothymol blue (BTB) as the indicator is employed in the above embodiment, phenol red (PR) can also be used as the indicator. When this indicator is used, it should be noted that wavelength regions in which the first absorbance and the second absorbance can be obtained are different from the case where the bromothymol blue (BTB) is employed as the indicator.

Up to this point, the case in which the rice grain is the target of measurement has been described. This measurement principle can also be applied to measurement of freshness of grains other than rice grains and beans.

The following are examples of results of freshness measurement according to the invention.

| VARIETY | | DATA VALUE | |
|---|---|---|---|
| | | Low temperature preservation | High temperature preservation |
| Wheat | Durum | 53 | 50 |
| Wheat | WW | 53 | 50 |
| Wheat | 1CW | 51 | 48 |
| Barley | - | 60 | 54 |
| Coffee Bean | Robusta | 68 | 61 |
| Corn | - | 46 | 46 |

In the above table, 5 gram of grains or beans which are targets of measurement and 10 gram of reagent are put into a test tube and stirred for one minute by the shaking machine. Then, after they are subjected to centrifugal separation for one minute by a small microcentrifuge, freshness of the target of measurement is measured. "Low-temperature preservation" shows a value obtained after preservation for 7 days at a temperature of 15°C and "High-temperature preservation" shows a value obtained after preservation for 7 days at a temperature of 60°C. A calibration curve on which freshness of unpolished new rice is 100 and freshness of rice having been preserved for one year at low temperature is 70 to 80 is created and freshness data values are calculated from this calibration curve. The above table shows freshness differences due to preservation environments of the grains and beans.

In the above table, the highest freshness data value obtained by the low-temperature preservation is 68 of coffee beans (unroasted beans) and the largest difference between the freshness data values obtained by the low-temperature preservation and the high-temperature preservation is 7 of the coffee beans. From this result, it can be said that grains and beans tend to deteriorate more when they are fresher (or, grains or beans that maintain highest freshness by low-temperature preservation will be affected more with regard to their freshness if preserved at high temperature, rather than low temperature.

Furthermore, it is also possible in the invention to measure respective absorbances at the above-mentioned two specific wavelengths absorbed by the reactive solution in the cell 3 by using a known spectrophotometer, instead of the freshness measurement device 1 shown in FIG. 1, to obtain a difference between the respective absorbances, and to judge freshness of grains and beans.

As described above, according to the invention, the absorbance (first absorbance) at the wavelength at which the absorbance of the light absorbed by the reactive solution reaches the peak and the absorbance (second absorbance) in the wavelength region in which the absorbance hardly varies are detected and the difference between the first and second absorbances is obtained. And it is judged that the freshness of grains or beans is higher when the difference between the first and second absorbances are larger. Therefore, it is possible to accurately judge the freshness of the grains and beans irrespective of the turbidity of a reactive solution.

## Claims

1. A method of measuring freshness of grains and beans, comprising:
putting grains including rice, wheat, barley and corn or beans including soybeans and coffee beans into a predetermined reagent solution and stirring the solution;
detecting a first absorbance of a reactive solution obtained at the stirring step, to which a light is irradiated, at a wavelength where an absorbance thereof reaches a peak, and a second absorbance in a wavelength region in which an absorbance hardly varies; and
obtaining the difference between the first and second absorbances detected at the absorbarice detecting step and judging the freshness of the grains or beans based on the difference.

2. The method of measuring freshness of grains and beans according to claim 1, wherein the reagent solution uses bromothymol blue or phenol red as an indicator.

3. An apparatus for measuring freshness of grains and beans, the apparatus comprising:
a cell for containing a reactive solution obtained by putting grains or beans which are targets of measurement into a reagent solution and stirring the solution;
a light source for applying a light to the cell containing the reactive solution;
spectroscope means for dividing the light which has passed through the cell into at least two lights;
a first filter for receiving one of the lights obtained by division by the spectroscope means and allowing a first wavelength to pass through itself and a second filter for receiving the other light and allowing a second wavelength to pass through itself;
a first photoreceptor for receiving a light of the first wavelength which has passed through the first filter and a second photoreceptor for receiving a light of the second wavelength which has passed through the second filter; and
arithmetical means for performing an operation to obtain the freshness of the brains or beans which are targets of measurement from an output of the first photoreceptor and an output of the second photoreceptor, wherein
the first wavelength is selected to a value where an absorbance of the reactive solution in the cell to which the light from the light source is irradiated becomes maximum locally and the second wavelength is selected to a value in a region in which the absorbance hardly varies.
